# NOUVEAU FASCICULE DE BREVET EUROPEEN

(11) **EP 1 457 196 B2**
(45) Date de publication et mention de la décision concernant l'opposition: **15.06.2011**
(45) Mention de la délivrance du brevet: 23.05.2007
(21) Numéro de dépôt: 04290574.5
(22) Date de dépôt: 03.03.2004
(51) Int. Cl.: A61K 8/87

(54) **Dispositif aérosol contenant une composition cosmétique comprenant un polyuréthane et un agent propulseur comprenant du diméthyléther et au moins un hydrocarbure**
Aerosol-Vorrichtung, die eine, mit einem Polyurethan und einem Treibmittel bestehend aus Dimethylether und mindestens einem Kohlenwasserstoff bereitete kosmetische Zubereitung enthält
Aerosol device containing a cosmetic composition with a polyurethane and a propellant made of dimethylether and at least one hydrocarbon

(30) Priorité: 11.03.2003 FR 0303000
(43) Date de publication de la demande: 15.09.2004
(73) Titulaire: L'Oréal, 75008 Paris (FR)
(72) Inventeur: Le Bourhis, Francois, 93300 Aubervilliers (FR); Pataut, Francoise, 75017 Paris (FR)
(74) Mandataire: Dossmann, Gérard

(56) Documents cités:
- EP-A- 0 796 611
- EP-A2- 0 937 451
- EP-A2- 0 938 889
- WO-A1-00/12051
- WO-A2-99/48462
- US-A- 5 626 840
- US-A1- 2001 051 135
- H. BERKHOUT: "Hydrocarbon/dimethyl ether mixtures in water based hairsprays, part 1." AEROSOL AND SPRAY REPORT, vol. 33, no. 6, 1994, pages 292-294,296-298,300-301, XP008025705 Germany
- H. BERKHOUT: "Hydrocarbon/dimethyl ether mixtures in water based hairsprays. Part 2." AEROSOL ANS SPRAY REPORT, vol. 33, no. 7-8, 1994, pages 385--389, XP008025707
- H. BERKHOUT: "Hydrocarbon/dimethyl ether mixtures in water based hairsprays. Part 3." AEROSOL AND SPRAY REPORT, vol. 33, no. 9, 1994, pages 449-452,454-455, XP008025709

## Description

La présente invention concerne un dispositif aérosol contenant une composition cosmétique comprenant dans un milieu cosmétiquement acceptable contenant de l'eau et au moins un solvant organique, au moins un polyuréthane selon l'invention, et un agent propulseur comprenant du diméthyléther et au moins un hydrocarbure. L'invention vise également un procédé pour la mise en forme ou le maintien de la coiffure dans lequel ces dispositifs sont mis en oeuvre, ainsi que l'utilisation de ces dispositifs pour appliquer une laque.

Les compositions cosmétiques pour la mise en forme et/ou le maintien de la coiffure les plus répandues sur le marché de la cosmétique sont des compositions à pulvériser essentiellement constituées d'une solution le plus souvent alcoolique et d'un ou plusieurs composants, généralement des résines polymères, dont la fonction est de former des soudures entre les cheveux, appelés composants fixants, en mélange avec divers adjuvants cosmétiques. Cette solution est généralement conditionnée soit dans un récipient aérosol approprié mis sous pression à l'aide d'un agent propulseur, soit dans un flacon pompe.

On connaît de nombreux systèmes aérosols destinés à fixer les chevelures, ces systèmes contenant d'une part une phase liquide (ou jus) et d'autre part un agent propulseur. Ce dernier a pour fonction d'assurer une pression permettant la pulvérisation de la phase liquide, et son application sur les cheveux sous forme d'un nuage de gouttelettes dispersées.

Certaines formulations non aqueuses sont caractérisées par la génération d'un spray qualifié de doux. Ce terme recouvre un ensemble de caractéristiques sensorielles liées entre autres à la force du spray et à une signature acoustique réduite. Ces caractéristiques sont habituellement obtenues de différentes manières prises isolément ou non, telles que la pression réduite de la formulation ou son faible débit.

Dans le souci de diminuer à la valeur de 80% v/v la quantité de composés organiques volatils (COV) tels que les alcools et les hydrocarbures fluorés présents dans ce type de compositions à pulvériser, on introduit habituellement de 15 à 20% v/v d'eau dans les formulations. Pour des raisons de solubilité du milieu, cette présence d'eau dans la formule implique l'usage de diméthyléther comme agent propulseur avec pour conséquences une augmentation de la pression interne, du débit, de la force du spray et du bruit ce qui a pour conséquence l'obtention d'un spray qu'on ne peut plus qualifier de doux.

Se pose alors le problème d'obtenir des formulations caractérisées par la génération d'un spray doux contenant du diméthyléther comme gaz propulseur. Les solutions mises en oeuvre pour obtenir un spray doux avec ce type de formulation, et qui consistent par exemple à réduire le débit, ne donnent pas de résultat pleinement satisfaisant. Les qualités d'usage attendues se dégradent : par exemple le niveau de fixation obtenu devient significativement trop faible. L'introduction d'hydrocarbures en association avec le diméthyléther dans une formulation contenant des polymères fixants classiques ne résout pas non plus ce problème.

Des dispositifs aérosols utilisant comme agent propulseur du diméthyléther et contenant comme polymère fixant un polycondensat comprenant au moins un motif polyuréthane et/ou polyurée ont été décrits dans la demande FR A 2 782 635. Néanmoins, ces formulations ne permettent pas de générer un spray doux.

De même, la demande US 5 626 840 décrit une composition pour la fixation des cheveux comprenant un polyuréthane linéaire carboxylé, une base, un solvant ainsi que du diméthyléther ou un hydrocarbure comme agent propulseur. De la même manière, ce type de formulation ne permet pas de générer un spray qualifié de doux.

La demanderesse a trouvé de manière surprenante et avantageuse, que l'usage dans un milieu contenant de l'eau et au moins un solvant organique, d'un polyuréthane selon l'invention avec comme agent propulseur un mélange de diméthyléther et d'un ou plusieurs hydrocarbures permet l'obtention d'un spray doux, tout en conservant un bon niveau de fixation et de bonnes propriétés cosmétiques telles que la douceur ou le démêlage.

La présente invention a donc pour objet un dispositif aérosol contenant une composition cosmétique comprenant un polyuréthane selon l'invention, et un mélange de gaz propulseurs comprenant du diméthyléther et au moins un hydrocarbure.

Un autre objet de l'invention concerne un procédé pour la mise en forme ou le maintien de la coiffure comprenant la mise en oeuvre de ce dispositif aérosol.

Un autre objet de l'invention consiste en l'utilisation dudit dispositif pour appliquer une laque sur les cheveux.

D'autres objets, caractéristiques, aspects et avantages de l'invention apparaîtront encore plus clairement à la lecture de la description et des divers exemples qui suivent.

La présente invention concerne un dispositif aérosol contenant :
A. une composition cosmétique comprenant dans un milieu cosmétiquement acceptable contenant de l'eau et au moins un solvant organique, au moins un polyuréthane, comprenant au moins une séquence polysiloxane et son motif répétitif de base répond à la formule générale (VII) :

   - X' - P - X' - CO - NH - R - NH - CO - (VII)

   dans laquelle :
   - P est un segment polysiloxanique,
   - X' représente O et/ou NH, et
   - R est un radical divalent choisi parmi les radicaux alkylènes ramifiés ou non de type aromatique en C₆-C₂₀, aliphatique en C₁ à C₂₀, de préférence en C₁-C₆, cycloaliphatique en C₁ à C₂₀, de préférence en C₁-C₆, ces radicaux étant non substitués ou substitués par un ou plusieurs groupements halogène, alcoxy en C₁-C₄, aryle en C₆-C₃₀, en particulier phényle.
B. un agent propulseur, comprenant du diméthyléther et au moins un hydrocarbure en C₃-C₅.

L'agent propulseur représente de 20 à 70% en poids par rapport au poids total de l'ensemble des compositions contenues dans le dispositif aérosol.

Les hydrocarbures en C₃-C₅ selon l'invention sont choisis de préférence parmi le n-butane, l'isobutane, le propane, ou un mélange de ces hydrocarbures. De préférence, le n-butane sera utilisé.

Les polyuréthanes de l'invention sont des polyuréthanes fixants. Par polyuréthane fixant, on entend un polyuréthane dont la fonction est de conférer ou de maintenir une forme donnée à la coiffure.

Les polyuréthanes utilisés de préférence selon l'invention présentent un motif répétitif de base répondant à la formule générale (I) suivante :

- X'-B-X'-CO-NH-R-NH-CO- (I)

dans laquelle
- X' représente O et/ou NH,
- B est un radical hydrocarboné divalent, ce radical étant substitué ou non, et
- R est un radical divalent choisi parmi les radicaux alkylènes ramifiés ou non de type aromatique en C₆-C₂₀, aliphatique en C₁ à C₂₀, de préférence en C₁-C₆, cycloaliphatique en C₁ à C₂₀, de préférence en C₁-C₆, ces radicaux étant non substitués ou substitués par un ou plusieurs groupements halogène, alcoxy en C₁-C₄, aryle en C₆-C₃₀, en particulier phényle.

De préférence, le radical B est un radical divalent en C₁-C₃₀, de préférence C₂-C₁₀ et est porteur d'un groupement présentant une ou des fonctions carboxyliques et/ou une ou des fonctions sulfoniques, lesdites fonctions carboxyliques et/ou sulfoniques étant sous forme libre ou partiellement ou totalement neutralisées par une base minérale ou organique telle que les hydroxydes des métaux alcalins ou alcalinoterreux, l'ammoniaque et les alkylamines ou alcanolamines, les acides aminés organiques. De préférence B est le radical divalent issu de l'acide diméthylolpropionique.

Le radical R est avantageusement choisi parmi les radicaux répondant aux formules suivantes :

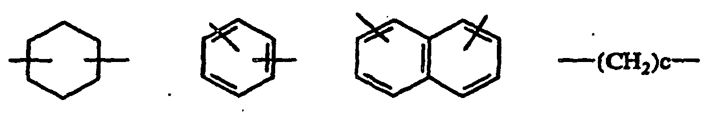

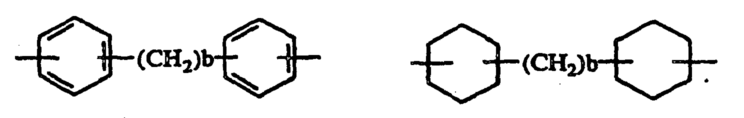

Dans lesquelles b est un nombre entier compris entre 0 et 3, et c un nombre entier compris entre 1 et 20, de préférence compris entre 2 et 12.

En particulier, le radical R est choisi parmi les radicaux héxaméthylène, 4,4'-biphénylèneméthane, 2,4- et/ou 2,6-tolylène, 1,5-naphtylène, p-phénylène, méthylène-4,4bis-cyclohéxyle et le radical divalent dérivé de l'isophorone.

Selon la présente invention, les polyuréthanes fixant peuvent comporter des greffons silicones et des silicones à greffons hydrocarbonés. Ces différents composés peuvent être non ioniques, cationiques ou amphotères.

Un polyuréthane utilisé dans la présente invention comprend au moins une séquence polysiloxane et son motif répétitif de base répond à la formule générale (VII) :

- X' - P - X' - CO - NH - R - NH - CO - (VII)

dans laquelle :
- P est un segment polysiloxanique,
- X' représente O et/ou NH, et
- R est un radical divalent choisi parmi les radicaux alkylènes ramifiés ou non de type aromatique en C₆-C₂₀, aliphatique en C₁ à C₂₀, de préférence en C₁-C₆, cycloaliphatique en C₁ à C₂₀, de préférence en C₁-C₆, ces radicaux étant non substitués ou substitués par un ou plusieurs groupements halogène, alcoxy en C₁-C₄, aryle en C₆-C₃₀, en particulier phényle.

Le radical R est avantageusement choisi parmi les radicaux répondant aux formules suivantes :

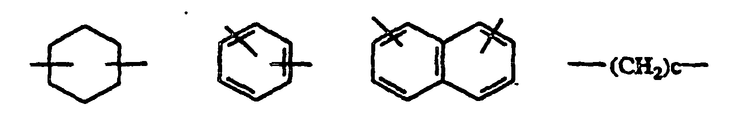

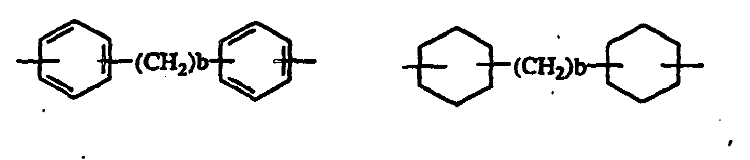

Dans lesquelles b est un nombre entier compris entre 0 et 3, et c un nombre entier compris entre 1 et 20, de préférence compris entre 2 et 12.

En particulier, le radical R est choisi parmi les radicaux héxaméthylène, 4,4'-biphénylèneméthane, 2,4- et/ou 2,6-tolylène, 1,5-naphtylène, p-phénylène, méthylène-4,4bis-cyclohéxyle et le radical divalent dérivé de l'isophorone.

Avantageusement, le segment polysiloxanique P répond à la formule générale (VIII) ci-après : 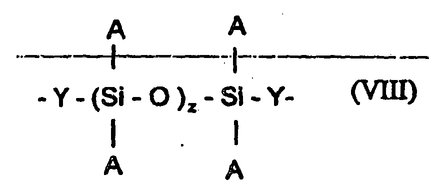 dans laquelle:
- les groupes A, qui peuvent être identiques ou différents, sont choisis parmi d'une part les groupes hydrocarbonés monovalents en C₁ à C₂₀ substantiellement exempts d'insaturation éthylénique et, d'autre part, les groupes aromatiques,
- Y représente un groupe hydrocarboné divalent, et
- z représente un nombre entier, choisi de telle sorte que la masse moléculaire moyenne du segment polysiloxane soit comprise entre 300 et 10 000.

En général, le groupe divalent Y est choisi parmi les groupes alkylènes de formule -(CH₂)ₐ- , dans laquelle a représente un nombre entier pouvant être compris entre 1 et 10.

Les groupes A peuvent être choisis parmi les groupes alkyle en C₁-C₁₈, en particulier les groupes méthyle, éthyle, propyle, isopropyle, butyle, pentyle, hexyle, octyle, décyle, dodécyle et octadécyle ; les groupes cycloalkyle, en particulier le groupe cyclohexyle ; les groupes aryle, notamment phényle et naphtyle ; les groupes arylalkyle, notamment benzyle et phényléthyle, ainsi que les groupes tolyle et xylyle.

De préférence, le polyuréthane de l'invention est anionique.

A titre d'exemples de polyuréthane fixant, on peut notamment citer le copolymère acide diméthylol-propionique/isophorone-diisocyanate/néopentylglycol/polyesterdiols/diamine siliconée (connu aussi sous le nom de polyuréthane-6, appellation INCI) vendu sous la marque Luviset® Si PUR A par la société BASF.

De préférence, le polymère utilisé est celui commercialisé sous la dénomination Luviset SI-PUR ZK 553-24 par la société BASF.

La composition cosmétique selon l'invention comprend de préférence le ou les polyuréthanes en une quantité comprise entre 0,5 et 20% en poids, de préférence entre 2 et 12% en poids par rapport au poids total de l'ensemble des compositions contenues dans le dispositif aérosol.

L'agent propulseur représente 20 à 70% en poids par rapport au poids total de l'ensemble des compositions contenues dans le dispositif aérosol.

L'agent propulseur contient de 1 à 30%, de préférence de 5 à 20% en poids d'hydrocarbure par rapport au poids total de l'ensemble des compositions contenues dans le dispositif aérosol.

Par ensemble des compositions contenues dans le dispositif aérosol, on entend au sens de la présente invention la composition cosmétique A et l'agent propulseur B.

Le milieu cosmétiquement acceptable est un milieu contenant de l'eau et au moins un solvant organique.

Par solvant organique, on entend au sens de la présente invention un composé organique liquide à la température de 25°C et à la pression atmosphérique. De préférence, le composé organique est polaire.

De préférence, le solvant est un alcool. Celui-ci est notamment choisi parmi les alcools inférieurs en C₁-C₄ comme l'éthanol, l'isopropanol, le tertio-butanol, le n-butanol ; les polyols comme le propylèneglycol, les éthers de polyols et leurs mélanges, l'alcool particulièrement préféré étant l'éthanol.

Dans le milieu cosmétiquement acceptable, la proportion en eau peut être comprise entre 0,5 et 35% en poids par rapport au poids total de l'ensemble des compositions contenues dans le dispositif aérosol. La proportion en solvant organique du mélange est comprise entre 1 et 70% en poids, de préférence entre 15 et 65% en poids et encore plus préférentiellement entre 30 à 60% en poids par rapport au poids total de l'ensemble des compositions contenues dans le dispositif aérosol.

La composition cosmétique fixante selon l'invention peut contenir en outre au moins un adjuvant choisi parmi les autres polymères fixants non polyuréthane comme les silicones sous forme soluble, dispersée, micro-dispersée, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, le glycérol, les pigments minéraux et organiques, colorés ou non colorés, les colorants permanents ou temporaires, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums, les agents solubilisants du parfum (peptisant), les agents conservateurs, les agents anti-corrosion, et les actifs traitants.

L'homme de métier veillera à choisir les éventuels additifs et leur quantité de manière à ce qu'ils ne nuisent pas aux propriétés des compositions de la présente invention.

Ces additifs sont présents dans la composition cosmétique selon l'invention en une quantité allant de 0 à 20 % en poids par rapport au poids total de la composition cosmétique.

Les compositions conformes à l'invention sont utilisées en tant que compositions de mise en forme et/ou de maintien des cheveux.

La présente invention concerne également un procédé capillaire pour la mise en forme ou le maintien de la coiffure, comprenant la mise en oeuvre du dispositif décrit ci-dessus.

Un autre objet de l'invention concerne l'utilisation du dispositif pour appliquer une laque sur les cheveux par vaporisation de son contenu.

Les exemples suivants illustrent la présente invention et ne doivent être considérés en aucune manière comme limitant l'invention.

### Exemple 1 :

La demanderesse a réalisé un dispositif conforme à l'invention comprenant la formulation 1 suivante:

| | |
|---|---|
| Résine LUVISET SI-PUR ZK 553-24 (fournisseur BASF) (exprimé en poids de polymère) | 6,00 |
| PARSOL MCX (éthylhéxyl méthoxycinnamate (fournisseur ROCHE VITAMINS) | 0,05 |
| Eau déminéralisée (contenue dans le polymère sous sa forme commerciale) | 12 |
| Eau déminéralisée formulaire | 3 |
| Parfum | 0,10 |
| n-butane | 15 |
| diméthyléther | 25 |
| Alcool éthylique absolu qsq | 100 |

### Exemple 2 :

La demanderesse a réalisé un dispositif conforme à l'invention comprenant la formulation 2 :

| | |
|---|---|
| Résine LUVISET SI-PUR ZK 553-22 (fournisseur BASF) (exprimé en poids de polymère) | 6,00 |
| PARSOL MCX (éthylhéxyl méthoxycinnamate (fournisseur ROCHE VITAMINS) | 0,05 |
| Eau déminéralisée (contenue dans le polymère sous sa forme commerciale) | 12 |
| Eau déminéralisée formulaire | 3 |
| Parfum | 0,10 |
| n-butane | 15 |
| diméthyléther | 25 |
| Alcool éthylique absolu qsq | 100 |

Les formulations 1 et 2 conditionnées dans un dispositif aérosol et pulvérisées sur les cheveux permettent d'obtenir un spray qualifié de doux et confèrent aux cheveux un très bon maintien.

## Revendications

1. Dispostif aérosol contenant
A. une composition cosmétique comprenant dans un milieu cosmétiquement acceptable contenant de l'eau et au moins un solvant organique, au moins un polyuréthane, comprenant au moins un motif répétitif de base répondant à la formule générale (VII) :
-X'-P-X'-CO-NH-R-NH-CO- (VII)
dans laquelle :
- P est un segment polysiloxanique,
- X' représente O et/ou NH, et
- R est un radical divalent choisi parmi les radicaux alkylènes ramifiés ou non de type aromatique en C₈-C₂₀, aliphatique en C₁ à C₂₀, de préférence en C₁-C₆, cycloaliphatique en C₁ à C₂₀, de préférence en C₁-C₆, ces radicaux étant non substitués ou substitué par un ou plusieurs groupements halogène, alcoxy en C₁-C₄, aryle en C₆-C₃₀.
B. et un agent propulseur comprenant du diméthyléther et au moins un hydrocarbure en C₃-C₅, l'agent propulseur représentant de 20 à 70% en poids par rapport au poids total de l'ensemble des compositions contenues dans le dispositif aérosol.

2. Dispositif selon la revendication 1, **caractérisé en ce que** le ou les hydrocarbures sont choisis parmi le n-butane, l'isobutane, le propane, ou un mélange de ces hydrocarbures.

3. Dispositif selon la revendication 2, **caractérisé en ce que** l'hydrocarbure est le n-butane.

4. Dispositif selon l'une quelconque des revendications 1 à 3, **caractérisé en ce que** le polyuréthane selon l'invention présente un motif répétitif de base répondant la formule générale (I) suivante :
-X'-B-X'-CO-NH-R-NH-CO- (I)
dans laquelle
- X' représente O et/ou NH,
- B est un radical hydrocarboné divalent, ce radical étant substitué ou non, et
- R est un radical divalent choisi parmi les radicaux alkylènes ramifiés ou non, de type aromatique en C₈-C₂₀, aliphatique sen C₁ à C₂₀, de préférence en C₁-C₆, cycloaliphatique en C₁ à C₂₀, de préférence en C₁-C₆, ces radicaux étant non substitués ou substitué par
un ou plusieurs groupements halogène, alcoxy en C₁-C₄, aryle en C₆-C₃₀, de préférence phényle.

5. Dispositif selon la revendication 4, **caractérisé en ce que** le radical B est un radical hydrocarboné divalent en C₁ à C₃₀.

6. Dispositif selon la revendication 4 ou 5, **caractérisé en ce que** le radical R est choisi parmi dans le groupe comprenant les radicaux héxaméthylène, 4,4'-biphénylèneméthane, 2,4- et/ou 2,6-tolylène, 1,5-naphtylène, p-phénylène, méthylène-4,4 bis-cyclohéxyle et le radical divalent dérivé de l'isophorone.

7. Dispositif selon l'une quelconque des revendications 1 à 6, **caractérisé en ce que** le polyuréthane est anionique.

8. Dispositif selon l'une quelconque des revendications précédentes, **caractérisé en ce que** la composition cosmétique contient de 0,5 à 20%, et de préférence de 2 à 12% de polyuréthane par rapport au poids total de l'ensemble des compositions contenues dans le dispositif aérosol.

9. Dispositif selon l'une quelconque des revendications précédentes, **caractérisé en ce que** l'agent propulseur contient de 1 à 30%, et de préférence de 5 à 20% d'hydrocarbures par rapport au poids total de l'ensemble des compositions contenues dans le dispositif aérosol.

10. Dispositif selon l'une quelconque des revendications précédentes, **caractérisé en ce que** le solvant organique est un alcool.

11. Dispositif selon la revendication 10, **caractérisé en ce que** l'alcool est choisi parmi les alcools en C₁-C₄, les polyols, les éthers de polyols et leurs mélanges.

12. Dispositif selon la revendication 11, **caractérisé en ce que** l'alcool est l'éthanol.

13. Dispositif selon l'une quelconque des revendications précédentes, **caractérisé en ce que** la composition cosmétique contient de 0,5 à 35% d'eau en poids par rapport au poids total de l'ensemble des compositions contenues dans le dispositif aérosol.

14. Dispositif selon l'une quelconque des revendications précédentes, **caractérisé en ce que** la composition cosmétique contient de 1 à 70% en poids d'alcool en C₁-C₄, de préférence de 15 à 65% et encore plus préférentiellement de 30 à 60% en poids par rapport au poids total de l'ensemble des compositions contenues dans le dispositif aérosol.

15. Dispositif selon rune quelconque des revendications précédentes, **caractérisé par le fait que** la composition coiffante comprend un adjuvant choisi parmi les autres polymères fixants non polyuréthane, les silicones sous forme soluble, dispersée, micro-dispersée, les agents tensio-actifs non-ioniques, anioniques, cationiques et amphotères, les céramides et pseudo-céramides, les vitamines et pro-vitamines dont le panthénol, les huiles végétales, animales, minérales et synthétiques, les cires autres que les céramides et pseudo-céramides, les filtres solaires hydrosolubles et liposolubles, siliconés ou non siliconés, le glycérol, les pigments minéraux et organiques, colorés ou non colorés, les colorants permanents ou temporaires, les agents nacrants et opacifiants, les agents séquestrants, les agents plastifiants, les agents solubilisants, les agents acidifiants, des agents alcalinisants, les agents épaississants minéraux et organiques, les agents anti-oxydants, les hydroxyacides, les agents de pénétration, les parfums, les agents solubilisants du parfum, les agents conservateurs, les agents anti-corrosion, et les actifs traitrants.

16. Procédé de traitement cosmétique pour la mise en forme ou le maintien de la coiffure, **caractérisé par le fait qu'**il comprend la mise en oeuvre d'un dispositif conforme à l'une quelconque des revendications 1 à 15.

17. Utilisation du dispositif selon l'une quelconque des revendications 1 à 15, pour appliquer une laque sur les cheveux par vaporisation de son contenu.

## Claims

1. Aerosol device containing:
A. a cosmetic composition comprising, in a cosmetically acceptable medium containing water and at least one organic solvent, at least one polyurethane comprising at least one base repeating unit corresponding to the general formula (VII):
—X'—P—X'—CO—NH—R—NH—CO (VII)
in which:
- P is a polysiloxane segment,
- X' represents O and/or NH, and
R is a divalent radical chosen from branched or unbranched alkylene radicals of C₆-C₂₀ aromatic type, C₁-C₂₀ and preferably C₁-C₆ aliphatic type and C₁-C₂₀ and preferably C₁-C₆ cycloaliphatic type, these radicals being unsubstituted or substituted with one or more halogen, C₁-C₄ alkoxy and C₆-C₃₀ aryl groups, and
B. a propellant comprising dimethyl ether and at least one C₃-C₅ hydrocarbon, the propellant representing from 20% to 70% by weight relative to the total weight of all of the compositions contained in the aerosol device.

2. Device according to Claim 1, **characterized in that** the hydrocarbon(s) is (are) chosen from n-butane, isobutane and propane, or a mixture of these hydrocarbons.

3. Device according to Claim 2, **characterized in that** the hydrocarbon is n-butane.

4. Device according to any one of Claims 1 to 3, **characterized in that** the polyurethane according to the invention contains a base repeating unit corresponding to the general formula (I) below:
-X'-B-X'-CO-NH-R-NH-CO (I)
in which
- X' represents O and/or NH,
- B is a divalent hydrocarbon-based radical, this radical being substituted or unsubstituted, and
- R is a divalent radical chosen from branched or unbranched alkylene radicals of C₆-C₂₀ aromatic type, C₁-C₂₀ and preferably C₁-C₆ aliphatic type and C₁-C₂₀ and preferably C₁-C₆ cycloaliphatic type, these radicals being unsubstituted or substituted with one or more halogen, C₁-C₄ alkoxy or C₆-C₃₀ aryl, preferably phenyl, groups.

5. Device according to Claim 4, **characterized in that** the radical B is a divalent C₁-C₃₀ hydrocarbon-based radical.

6. Device according to Claim 4 or 5, **characterized in that** the radical R is chosen from the group comprising hexamethylene, 4,4'-biphenylenemethane, 2,4- and/or 2,6-tolylene, 1,5-naphthylene, p-phenylene and methylene-4,4-bis-cyclohexyl radicals and the divalent radical derived from isophorone.

7. Device according to any one of Claims 1 to 6, **characterized in that** the polyurethane is anionic.

8. Device according to any one of the preceding claims, **characterized in that** the cosmetic composition contains from 0.5% to 20% and preferably from 2% to 12% of polyurethane relative to the total weight of all of the compositions contained in the aerosol device.

9. Device according to any one of the preceding claims, **characterized in that** the propellant contains from 1% to 30% and preferably from 5% to 20% of hydrocarbons relative to the total weight of all the compositions contained in the aerosol device.

10. Device according to any one of the preceding claims, **characterized in that** the organic solvent is an alcohol.

11. Device according to Claim 10, **characterized in that** the alcohol is chosen from C₁-C₄ alcohols, polyols and polyol ethers, and mixtures thereof.

12. Device according to Claim 11, **characterized in that** the alcohol is ethanol.

13. Device according to any one of the preceding claims, **characterized in that** the cosmetic composition contains from 0.5% to 35% water by weight relative to the total weight of all of the compositions contained in the aerosol device.

14. Device according to any one of the preceding claims, **characterized in that** the cosmetic composition contains from 1% to 70% by weight of C₁-C₄ alcohol, preferably from 15% to 65% and even more preferably from 30% to 60% by weight, relative to the total weight of all of the compositions contained in the aerosol device.

15. Device according to any one of the preceding claims, **characterized in that** the styling composition comprises an adjuvant chosen from the other non-polyurethane fixing polymers, silicones in soluble, dispersed or microdispersed form, nonionic, anionic, cationic and amphoteric surfactants, ceramides and pseudoceramides, vitamins and provitamins including panthenol, plant, animal, mineral and synthetic oils, waxes other than ceramides and pseudoceramides, watersoluble and liposoluble, silicone or non-silicone sunscreens, glycerol, mineral and organic, coloured or uncoloured pigments, permanent or temporary dyes, nacreous agents and opacifiers, sequestrants, plasticizers, solubilizing agents; acidifying agents, basifying agents, mineral and organic thickeners, antioxidants, hydroxy acids, penetrants, fragrances, fragrance solubilizers, preserving agents, anticorrosion agents and treatment agents.

16. Cosmetic treatment process for shaping or holding the hairstyle, **characterized in that** it comprises the use of a device in accordance with any one of Claims 1 to 15.

17. Use of the device according to any one of Claims 1 to 15, to apply a lacquer to the hair by vaporizing its contents.

## Patentansprüche

1. Aerosolvorrichtung enthaltend:
A. eine kosmetische Zusammensetzung, die in einem kosmetisch akzeptablen Medium, das Wasser und mindestens ein organisches Lösungsmittel enthält, mindestens ein Polyurethan mindestens eine wiederkehrende Grundeinheit aufweist, die der allgemeinen Formel (VII) entspricht:
-X'-P-X'-CO-NH-R-NH-CO (VII)
worin bedeuten:
- P ein Polysiloxansegment,
- X' O und/oder NH, und
R eine zweiwertige Gruppe, die unter den verzweigten oder unverzweigten Alkylengruppen vom aromatischen Typ mit 6 bis 20 Kohlenstoffatomen, aliphatischen Typ mit 1 bis 20 Kohlenstoffatomen und vorzugsweise 1 bis 6 Kohlenstoffatomen, cycloaliphatischen Typ mit 1 bis 20 Kohlenstoffatomen und vorzugsweise 1 bis 6 Kohlenstoffatomen ausgewählt ist, wobei diese Gruppen unsubstituiert vorliegen oder mit einer oder mehreren Gruppen Halogen, C₁-₄-Alkoxy, C₆-₃₀-Aryl substituiert sind, enthält,
B. und ein Treibmittel, das Dimethylether und mindestens einen C₃-₅-Kohlenwasserstoff umfasst, wobei das Treibmittel 20 bis 70 Gew. -%, bezogen auf das Gesamtgewicht der gesamten, in der Aerosolvorrichtung enthaltenen Zusammensetzungen, ausmacht.

2. Vorrichtung nach Anspruch 1, **dadurch gekennzeichnet, dass** der oder die Kohlenwasserstoffe unter *n*-Butan, Isobutan, Propan oder einem Gemisch dieser Kohlenwasserstoffe ausgewählt sind.

3. Vorrichtung nach Anspruch 2, **dadurch gekennzeichnet, dass** der Kohlenwasserstoff das *n*-Butan ist.

4. Vorrichtung nach einem der Ansprüche 1 bis 3, **dadurch gekennzeichnet, dass** das erfindungsgemäße Polyurethan wiederkehrende Grundeinheiten aufweist, die der folgenden allgemeinen Formel (I) entsprechen:
-X'-B-X'-CO-NH-R-NH-CO (I)
worin bedeuten:
X' O und/oder NH,
B eine zweiwertige Kohlenwasserstoffgruppe, wobei diese Gruppe substituiert ist oder unsubstituiert vorliegt,
und
R eine zweiwertige Gruppe, die unter den verzweigten oder unverzweigten Alkylengruppen vom aromatischen Typ mit 6 bis 20 Kohlenstoffatomen, aliphatischen Typ mit 1 bis 20 Kohlenstoffatomen und vorzugsweise 1 bis 6 Kohlenstoffatomen, cycloaliphatischen Typ mit 1 bis 20 Kohlenstoffatomen und vorzugsweise 1 bis 6 Kohlenstoffatomen ausgewählt ist, wobei diese Gruppen unsubstituiert vorliegen oder mit einer oder mehreren Gruppen Halogen, C₁-₄-Alkoxy, C₆-₃₀-Aryl und vorzugsweise Phenyl substituiert sind.

5. Vorrichtung nach Anspruch 4, **dadurch gekennzeichnet, dass** die Gruppe B eine zweiwertige Kohlenwasserstoffgruppe mit 1 bis 30 Kohlenstoffatomen ist.

6. Vorrichtung nach Anspruch 4 oder 5, **dadurch gekennzeichnet, dass** die Gruppe R unter den Gruppen Hexamethylen, 4,4'-Biphenylenmethan, 2,4- und/oder 2,6-Toluylen, 1,5-Naphthylen, p-Phenylen, Methylen-4,4-bis-cyclohexyl und der zweiwertigen, von Isophoron abgeleiteten Gruppe ausgewählt ist.

7. Vorrichtung nach einem der Ansprüche 1 bis 6, **dadurch gekennzeichnet, dass** das Polyurethan anionisch ist.

8. Vorrichtung nach einem der vorhergehenden Ansprüche, **dadurch gekennzeichnet, dass** die kosmetische Zusammensetzung 0,5 bis 20% und vorzugsweise 2 bis 12% Polyurethan, bezogen auf das Gesamtgewicht der gesamten, in der Aerosolvorrichtung enthaltenen Zusammensetzungen, enthält.

9. Vorrichtung nach einem der vorhergehenden Ansprüche, **dadurch gekennzeichnet, dass** das Treibmittel 1 bis 30% und vorzugsweise 5 bis 20% Kohlenwasserstoffe, bezogen auf das Gesamtgewicht der gesamten, in der Aerosolvorrichtung enthaltenen Zusammensetzungen, enthält.

10. Vorrichtung nach einem der vorhergehenden Ansprüche, **dadurch gekennzeichnet, dass** das organische Lösungsmittel ein Alkohol ist.

11. Vorrichtung nach Anspruch 10, **dadurch gekennzeichnet, dass** der Alkohol unter den C₁-₄-Alkoholen, Polyolen, Polyolethern und deren Gemischen ausgewählt ist.

12. Vorrichtung nach Anspruch 11, **dadurch gekennzeichnet, dass** es sich bei dem Alkohol um Ethanol handelt.

13. Vorrichtung nach einem der vorhergehenden Ansprüche, **dadurch gekennzeichnet, dass** die kosmetische Zusammensetzung 0,5 bis 35 Gew.-% Wasser, bezogen auf das Gesamtgewicht der gesamten, in der Aerosolvorrichtung enthaltenen Zusammensetzungen, enthält.

14. Vorrichtung nach einem der vorhergehenden Ansprüche, **dadurch gekennzeichnet, dass** die kosmetische Zusammensetzung 1 bis 70 Gew.-% C₁-₄-Alkohol und vorzugsweise 15 bis 65% und noch bevorzugter 30 bis 60 Gew.-%, bezogen auf das Gesamtgewicht der gesamten, in der Aerosolvorrichtung enthaltenen Zusammensetzungen, enthält.

15. Vorrichtung nach einem der vorhergehenden Ansprüche, **dadurch gekennzeichnet, dass** die Zusammensetzung für die Haarbehandlung einen Zusatzstoff enthält, der unter den fixierenden Polymeren, bei denen es sich nicht um Polyurethan handelt, Siliconen in gelöster, dispergierter und mikrodispergierter Form, nichtionischen, anionischen, kationischen und amphoteren grenzflächenaktiven Stoffen, Ceramiden und Pseudoceramiden, Vitaminen und Provitaminen und darunter Panthenol, pflanzlichen Ölen, tierischen Ölen, Mineralölen und synthetischen Ölen, Wachsen, die von Ceramiden und Pseudoceramiden verschieden sind, wasserlöslichen und fettlöslichen, siliconierten oder nicht siliconierten Sonnenschutzfiltern, Glycerin, mineralischen und organischen Pigmenten, die farbig oder nicht farbig sind, dauerhaften oder temporären Farbstoffen, Perlglanzmitteln und Trübungsmitteln, Maskierungsmitteln, Weichmachern, Solubilisierungsmitteln, Ansäuerungsmitteln, Alkalisierungsmitteln, anorganischen und organischen Verdickungsmitteln, Antioxidantien, Hydroxysäuren, Penetrationsmitteln, Parfums, Solubilisierungsmitteln für das Parfum, Konservierungsmitteln, Korrosionsschutzmitteln und behandelnden Wirkstoffen ausgewählt ist.

16. Verfahren zur kosmetischen Behandlung für die Formgebung oder Festigung der Frisur, **dadurch gekennzeichnet, dass** es die Verwendung einer Vorrichtung nach einem der Ansprüche 1 bis 15 umfasst.

17. Verwendung der Vorrichtung nach einem der Ansprüche 1 bis 15, um einen Lack durch Versprühen ihres Inhalts auf die Haare aufzubringen.
